# EUROPEAN PATENT APPLICATION

(11) **EP 3 412 179 A1**
(43) Date of publication of application: **12.12.2018**
(21) Application number: 17746898.0
(22) Date of filing: 24.01.2017
(51) Int. Cl.: A47C 21/00

(54) **INTELLIGENT BED**

(30) Priority: 02.02.2016 CN 201620103798 U
(71) Applicant: Keeson Technology Corporation Limited, Jiaxing, Zhejiang 314016 (CN)
(72) Inventor: SHAN, Huafeng, Jiaxing Zhejiang 314016 (CN); CAO, Hui, Jiaxing Zhejiang 314016 (CN); YU, Qun, Jiaxing Zhejiang 314016 (CN)
(74) Representative: Viering, Jentschura & Partner mbB Patent- und Rechtsanwälte
(86) International application number: PCT/CN2017/072423
(87) International publication number: WO 2017/133578

(57) **Abstract**

An intelligent bed, comprising a bed body, an intelligent lamp (4) attached to the bed body, a sensor (1) provided on the bed body for acquiring monitoring data as to whether a user has left the bed, a main control box (2) for controlling the intelligent lamp, and a wireless communication module (3) for storing intelligent lamp information, receiving the monitoring data of the sensor (1) and conducting operation on the monitoring data, and then sending the intelligent lamp information corresponding to an operation result to the main control box (2) so as to control the intelligent lamp (4). The intelligent bed can realize a plurality of customized functions according to the set parameters of the intelligent lamp (4) under various user-customized modes.

## Description

### Field of Technology

The present application relates to an intelligent bed, in particular to an electric bed with an intelligent lighting function.

### Background Technology

When people get up in the middle of the night, they often bump due to darkness, which brings inconvenience to life. Turning on lights in the room will affect other people to rest.

Besides, in addition to sleeping, people also read, watch TV, meditate, etc. on bed, wherein brightness and color temperature of light for any of these activities are different, and the existing beds cannot adjust brightness and color temperature of light according to the activities a user wants to carry out.

In addition, people sometimes cannot fall into sleep properly because of higher pressure, and the existing beds cannot do anything to improve or help people improve their sleep quality.

Traditional lights cannot give good reading experience to people reading in bed and hurt eyes.

### Summary

It is to be noted that the purpose of the present application is to overcome one or more of the disadvantages that have been found in the prior art, and to provide an intelligent bed. The intelligent bed has customized scene modes to enhance personalized user experience. Specifically, the intelligent bed can monitor whether a user has left the bed, automatically turn on a bed light for the user who leaves the bed in the middle of the night, and automatically turn off the bed light after the user returns to the bed, preventing the user from bumping and not affecting others to rest. The intelligent bed can also provide reading, TV-watching, meditation and other scene modes to give users a better experience. The intelligent bed also provides different brightness and color temperature of lights using a bedside lamp, helping users with sleep disorders to improve sleep quality.

For this purpose, an intelligent bed with an intelligent lighting function is proposed according to the present application, which is realized by the following technical solutions:
An intelligent bed includes a bed body, an intelligent lamp attached to the bed body, a sensor provided on the bed body for acquiring monitoring data as to whether a user has left the bed, a main control box for controlling the intelligent lamp, the main control box including a processing module and controlling the intelligent lamp through the processing module, and a wireless communication module for storing intelligent lamp information, and receiving monitoring data of the sensor, conducting operation on the monitoring data, and then sending intelligent lamp information corresponding to the operation result to the main control box to control the intelligent lamp.

In the intelligent bed, when the sensor collects the monitoring data, the wireless communication module conducts operation based on the monitoring data detected by the sensor, and transmits a command to the main control box when the operation result is obtained, so as to control the intelligent lamp to enter the corresponding mode. When the sensor detects a change in the monitoring data, the wireless communication module sends a command to the main control box according to the change in the operation result, and controls the intelligent lamp to change the mode. Therefore, the intelligent bed can independently control the state of the intelligent lamp to adapt to different needs of the user, and the user can set personalized intelligent lamp information through the intelligent terminal according to personal preferences.

Further, the intelligent lamp information is transmitted by the intelligent terminal to the wireless communication module by means of wireless transmission. The intelligent bed sets the intelligent lamp information through the intelligent terminal. The intelligent terminal transmits the set intelligent lamp information to the wireless communication module by wireless transmission for storage. When the sensor monitors parameters, the wireless communication module conducts operation based on the parameters detected by the sensor, and, when the operation result is received, transmits the corresponding intelligent lamp information in the form of a control signal to the main control box to control the intelligent lamp to enter the corresponding mode. Since data is transmitted by the wireless communication module, the processing module can be disposed at any position without being limited to being disposed in the bed body, and in particular can be disposed in a remote server, thereby reducing the number of components carried by the intelligent bed itself, and thus reducing production costs of the intelligent bed.

Further, the intelligent bed further includes a plurality of movable bed planks driven by a driver. The driver is electrically connected to the main control box, and drives the movable bed planks to move according to the control signal of the main control box so that a user can set personalized information of the movable bed plank through the intelligent terminal according to personal preferences. That is, the movable bed plank can be adjusted to a specific angle according to a user's personal preferences.

Further, the intelligent lamp is provided at the lower part of the bed body, so that when people get up in the middle of the night, the intelligent bed can automatically sense that the user is leaving the bed, and turn on the intelligent lamp to provide illumination for the user who has left the bed without affecting the others to sleep.

Further, the intelligent lamp includes a deformable support, and the support extends from the bed body so that the user can fix the intelligent lamp to any desired position according to personal preferences.

Further, the sensor includes a thin film pressure sensor provided on the bed body to sense whether a user is on the bed.

Further, the sensor includes an infrared sensor provided along a periphery of the bed body to sense whether the user has left the bed.

Further, the sensor is a wearable device to obtain a user's location information, physical information, and the like without increasing bed body component. At the same time, the sensor only collects data from the user wearing the wearable device and thus does not affect other people.

Further, the sensor and the intelligent terminal are the same element. By this setting, the hardware device of the intelligent bed can be simplified, so that the user can adjust the set parameters any time.

Further, the intelligent lamp information includes at least trigger thresholds of a plurality of scene modes and parameters of brightness and color temperature of the plurality of scene modes. The scene modes include at least a reading mode, a TV-watching mode, a meditation mode, an assisted sleeping mode, and the brightness and color temperature of the lights in these modes to enhance personalized user experience.

Further, the intelligent bed further includes a timer electrically connected to the processing module. The processing module conducts operation based on a time sent by the timer to determine a control signal to be sent to the intelligent lamp to enhance freedom of customized setting.

Further, the intelligent terminal is a mobile phone or a tablet computer, so that the intelligent lamp information can be set anytime and anywhere.

With the above arrangement, the intelligent bed of the present application can combine adjustment of the intelligent lamp and adjustment of the movable bed planks in the scene modes to achieve the most comfortable state of the user. The sensor of the present application can detect whether a user is going to or leaving the bed, and can also detect the physical parameters of the user. Combined with the above parameters, scene modes can be set by setting parameters to improve the user experience. In particular, the sensor and the intelligent terminal can adopt the same element, so hardware installed in the intelligent bed can be greatly reduced. Therefore, the intelligent bed can monitor the user and realize customized functions according to the user-customized set parameters, which especially improves user experience.

### Brief Description of the Drawings

It should be understood that in the present application, all features, modifications, and/or embodiments may be combined in various combinations, except in the cases of obvious contradictions and incompatibilities.

By reading the following non-limiting illustrative embodiments, and in conjunction with the drawings, other features and advantages of the present application will become apparent. In the figures:
- Figure 1 is a schematic structural view showing an intelligent bed with an intelligent lighting function according to the present application; and
- Figure 2 is a flow chart showing the control steps of the system of Figure 1.

### Detailed Description

It should be understood that the abovementioned drawings are not drawn to actual scale, but are merely schematic representations of various preferred features for illustrating the basic principles of the present application. The design features disclosed in the present application, such as size, orientation, position, and shape, are determined based on specific applications and use environments.

The present application will be described in detail below with reference to the embodiments and the accompanying drawings. In these figures, the same reference numerals are used to refer to the same or equivalent elements of the present application in the drawings.

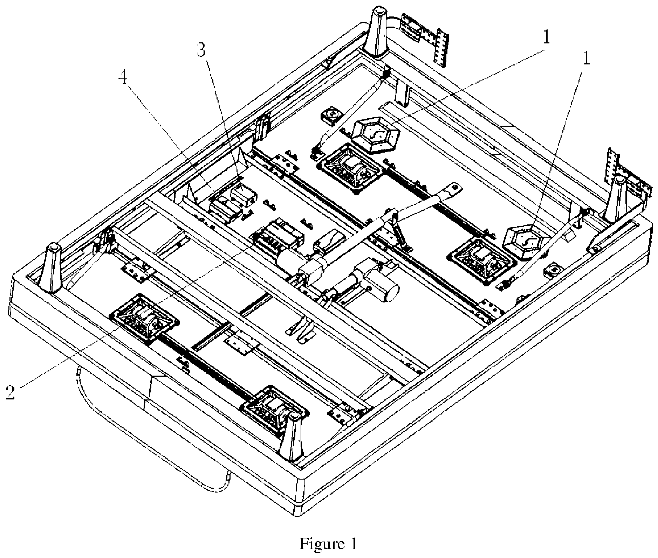

With reference to Figure 1, an intelligent bed with an intelligent lighting function according to the present application, in particular an electric bed with an intelligent lighting function is shown.

The electric bed can be any existing electric beds, and generally includes a bed body, a movable bed plank, a driver and a main control box 2. One end of the driver is hinged to the bed body and another end of the driver is hinged to the movable bed plank. Usually the movable bed plank is further mechanically connected to the bed body by a connecting rod. The main control box is electrically connected to the driver to control the driver to move the movable bed plank so that the movable bed plank is displaced relative to the bed body, such as to raise or lower the movable bed plank to provide a user with the most comfortable position.

The electric bed also includes an intelligent lamp 4, a sensor 1 and a wireless communication module 3, and a processing module, wherein the processing module is integrated into the main control box.

The intelligent lamp can be any of the existing intelligent lamps. Of course, ordinary lamps can also be used. The intelligent lamp is electrically connected to the main control box to adjust to different light brightness, light color, light frequency and the like according to the control of the main control box. The intelligent lamp can be mounted to different positions according to different needs. For example, the intelligent lamp can be mounted on bedside to meet reading needs, or the intelligent lamp can be mounted to the lower part of the bed body to meet lighting needs at nighttime. In addition, according to different needs, the intelligent lamp can also include a deformable support. The support extends from the bed body and can be adjusted and fixed to any position according to a user's preferences.

The sensor includes a pressure sensor mounted on the bed plank or mattress or bed body or bed body support, and can detect whether a user is going to bed or leaving the bed. In some embodiments, the sensor further includes an element for detecting the user's physical parameters.

As a modification, the sensor further includes an infrared sensor mounted along a periphery of the bed body to further accurately detect whether a user is going to bed or leaving the bed.

As a modification, the sensor can also be a wearable device so as to record physical parameters of a user after falling asleep, and at the same time can also record the distance between the user and the bed body, which further increases accuracy of the detection results.

The wireless communication module is electrically connected to the main control box, the sensor and the processing module, and is capable of wirelessly communicating with an intelligent terminal. The wireless communication module is connected with the intelligent terminal by means of wireless connection such as Wifi, Bluetooth, infrared, Zigbee, 2G, 3G, 4G or the Internet, and receives and saves intelligent lamp information sent by the intelligent terminal as set parameters of the intelligent lamp in different scene modes.

The intelligent lamp information includes at least trigger thresholds of a plurality of scene modes and parameters such as brightness and color temperature of the intelligent lamp in the set plurality of scene modes. Preferably, the set parameters further include time and a user's physical parameters.

The scene modes include at least a reading mode, a TV-watching mode, a meditation mode, an assisted sleeping mode, and a getting up at nighttime mode. A user can select a desired scene mode and set specific parameters through an operation interface of the intelligent terminal, and then the intelligent terminal transmits the same to the wireless communication module of the electric bed by wireless means. Wherein, the intelligent terminal can be a mobile phone or a tablet computer.

The wireless communication module receives the intelligent lamp information sent from the intelligent terminal. After collecting the corresponding monitoring data, the sensor conducts operation on the monitoring data. After acquiring the operation results, the wireless communication module sends a control signal to the main control box. The control signal includes adjusting the intelligent lamp to a specified mode to adjust brightness and color temperature emitted by the intelligent lamp.

In one embodiment, the sensor and the intelligent terminal are the same element, i.e., a wearable device for monitoring human physical parameters. In this way, the element can simultaneously achieve two functions, that is, it can send set parameters to the wireless communication module and send monitoring data to the wireless communication module.

In one embodiment, the electric bed further includes a timer electrically connected to the processing module in the main control box. The set parameters further include time-related parameters. The main control box compares parameters of the timer with the time-related parameters in the set parameters to determine a control signal to be sent to the intelligent lamp.

The operation process of the electric bed will be described below by way of example.

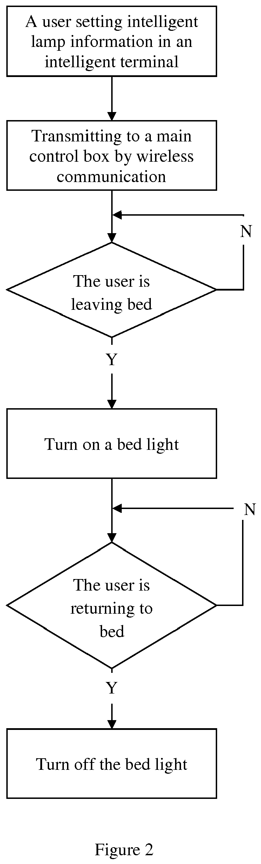

As shown in Figure 2, a flow chart of the control steps of an electric bed system of the present application controlled by an intelligent terminal is shown.

A user sets intelligent lamp information, including brightness and color temperature of the light, and trigger thresholds in scene modes such as reading, watching TV, meditation, assisted sleeping, and getting up at nighttime, through an intelligent terminal.

The intelligent terminal transmits the set intelligent lamp information to the wireless communication module by wireless transmission for storage, and sends the same to the wireless communication module when the sensor detects the corresponding trigger thresholds are reached. The wireless communication module itself calculates an operation result and sends a command to the main control box to control the intelligent lamp to turn on the corresponding function. When the sensor detects the trigger thresholds are not reached, the wireless communication module sends a signal to the main control box to control the intelligent lamp to turn off the corresponding function.

The user selects different scene modes through the intelligent terminal, including reading, watching TV, meditation, assisted sleeping, etc., and transmits a command to the wireless communication module by means of wireless communication. The wireless communication module sends the command to the main control box. The main control box controls the intelligent lamp by wired means.

Wherein, the user can also set information of the movable bed plank through the intelligent terminal, including an elevation angle of the movable bed plank and a trigger threshold in each scene mode.

Taking the getting up at nighttime mode as an example, the operation process is described. The user sets brightness and color temperature of the light in the getting up at nighttime scene mode through the intelligent terminal.

The intelligent terminal transmits the intelligent lamp information to the wireless communication module by wireless transmission for storage. When the sensor detects that the user is leaving the bed in the middle of the night, the wireless communication module sends a command to the main control box to control a bed light to be turned on. When the sensor detects that the user is returning to the bed, the wireless communication module sends a command to the main control box to control the bed light to be turned off.

The above embodiments are merely examples and do not limit the scope of the present application. Based on this, those skilled in the art can envision other embodiments that can achieve the same function within the scope of the claims of the present application.

Various embodiments and various modifications and improvements will be apparent to those skilled in the art. In particular, it should be understood that the above-described features, modifications, and/or embodiments of the present application may be combined with each other, except in the case of obvious contradictions or incompatibilities. All of these embodiments, as well as variations and modifications, are within the scope of the present application.

## Claims

1. An intelligent bed, comprising:
a bed body,
an intelligent lamp (4) attached to the bed body,
a sensor (1) provided on the bed body for acquiring monitoring data as to whether a user has left the bed,
a main control box (2) which comprises a processing module and controls the intelligent lamp through the processing module, and
a wireless communication module (3) for storing intelligent lamp information, and receiving the monitoring data of the sensor, conducting operation on the monitoring data, and then sending the intelligent lamp information corresponding to an operation result to the main control box to control the intelligent lamp.

2. The intelligent bed according to claim 1, wherein the intelligent lamp information is transmitted by an intelligent terminal to the wireless communication module by means of wireless transmission.

3. The intelligent bed according to claim 1 or 2, wherein the intelligent bed further comprises a plurality of movable bed planks driven by a driver, the driver is electrically connected to the main control box, and drives the movable bed planks to move according to a control signal of the main control box.

4. The intelligent bed according to claim 1 or 2, wherein the intelligent lamp is provided at a lower part of the bed body.

5. The intelligent bed according to claim 1 or 2, wherein the intelligent lamp comprises a deformable support, and the support extends from the bed body.

6. The intelligent bed according to claim 1 or 2, wherein the sensor comprises a thin film pressure sensor provided on the bed body.

7. The intelligent bed according to claim 1 or 2, wherein the sensor comprises an infrared sensor provided along a periphery of the bed body.

8. The intelligent bed according to claim 1 or 2, wherein the intelligent lamp information comprises at least trigger thresholds of a plurality of scene modes and parameters of brightness and color temperature of the plurality of scene modes.

9. The intelligent bed according to claim 1 or 2, wherein the intelligent bed further comprises a timer, the timer is electrically connected to the processing module, and the processing module conducts operation according to a time sent by the timer.

10. The intelligent bed according to claim 2, wherein the intelligent terminal is a mobile phone or a tablet computer.
